# FASCICULE DE BREVET EUROPEEN

(11) **EP 1 691 774 B1**
(45) Date de publication et mention de la délivrance du brevet: **03.11.2010**
(21) Numéro de dépôt: 04798987.6
(22) Date de dépôt: 02.11.2004
(51) Int. Cl.: A61K 8/18

(54) **COMPOSITION ORALE COMPRENANT UNE PREMIERE COMPOSITION (A) ET UNE SECONDE COMPOSITION (B) COMME PRODUIT DE COMBINAISON POUR UNE UTILISATION SEPAREE OU ETALEE DANS LE TEMPS, DANS LE TRAITEMENT COSMETIQUE DU CORPS HUMAIN**
ORALE ZUSAMMENSETZUNG MIT EINER ERSTEN ZUSAMMENSETZUNG (A) UND EINER ZWEITEN ZUSAMMENSETZUNG (B) ZUR VERWENDUNG ALS GEMISCHE ZUR GETRENNTEN ODER AUFEINANDERFOLGENDEN VERWENDUNG IN DER KOSMETISCHEN BEHANDLUNG DES MENSCHLICHEN KÖRPERS
ORAL COMPOSITION INCLUDING A FIRST COMPOSITION (A) AND A SECOND COMPOSITION (B) FOR USE AS A MIXTURE FOR SEPARATE OR CONSECUTIVE USE IN THE COSMETIC TREATMENT OF THE HUMAN BODY

(30) Priorité: 03.11.2003 FR 0312848
(43) Date de publication de la demande: 23.08.2006
(73) Titulaire: Laboratoires Macanthy S.A., 98000 Monaco (MC)
(72) Inventeur: Jacquet, Baudry, 3500 Hasselt (BE)
(74) Mandataire: Nargolwalla, Cyra
(86) Numéro de dépôt international: PCT/IB2004/003884
(87) Numéro de publication internationale: WO 2005/041916

(56) Documents cités:
- EP-A- 0 755 633
- EP-A- 1 344 516
- WO-A-02/15860
- WO-A-02/100329
- WO-A-03/086329
- FR-A- 2 809 596
- US-A- 5 976 568
- US-A1- 2002 098 253

## Description

La présente invention concerne une composition, destinée à une administration par voie orale, comprenant une première composition (a) et une seconde composition (b) comme produit de combinaison pour une utilisation séparée et étalée dans le temps, dans le traitement cosmétique du corps humain. La première composition (a) contient un extrait de thé vert, de la vitamine C, et éventuellement au moins un composé métallique choisi parmi le zinc, le chrome et un mélange de ceux-ci, et la seconde composition (b) contient au moins un composé métallique choisi parmi le fer, le cuivre, le chrome et un mélange de ceux-ci, ladite seconde composition (b) ne contenant pas de zinc. L'invention a également pour objet un procédé de traitement cosmétique du corps humain impliquant l'administration par voie orale d'un produit de combinaison comprenant une première composition (a) et une seconde composition (b) pour une utilisation séparée et étalée dans le temps.

La beauté, ou du moins l'apparence physique, est sans conteste, une préoccupation importante de nos contemporains et, encore aujourd'hui, plus particulièrement des femmes. Cette préoccupation esthétique concerne tout le corps, mais plus particulièrement la silhouette, la peau (notamment la peau du visage et des mains), et les phanères (ongles, cheveux).

Pour répondre à cette attente, de nombreux produits sont mis à la disposition des consommateurs tels que des produits cosmétiques, des médicaments et, plus récemment, des compléments alimentaires. En effet, des études scientifiques ont permis d'établir que l'apport nutritif, notamment l'apport en nutriments, en vitamines et minéraux, en acides gras insaturés et en certains extraits de plantes, pouvait avoir une grande influence sur l'aspect physique.

Il existe ainsi aujourd'hui de très nombreux produits (médicaments et compléments alimentaires) destinés à être administrés par voie orale et visant à améliorer ou corriger spécifiquement l'aspect de la peau, des phanères (cheveux et ongles) et de la silhouette. En pratique, les personnes soucieuses de leur aspect physique doivent prendre au moins trois produits différents, chacun ayant une posologie de deux à trois formes galéniques (comprimés, gélules, capsules molles, sachets de poudre, solution buvable,...) chaque jour. Ce sont donc six à neuf formes galéniques que ces personnes doivent ingérer chaque jour pour bénéficier des effets attendus. Outre le fait que ce nombre important de prises est très contraignant et favorise les oublis, le fait d'associer des produits n'ayant pas été conçus pour être utilisés simultanément entraîne des risques de surdosages ou d'incompatibilités entre les ingrédients.

Si l'on ajoute à cela le fait que pour préserver la jeunesse de son aspect physique ou pour améliorer celui-ci, il est nécessaire d'utiliser ces produits en continu ou, du moins sur de longues périodes, on comprend tout l'intérêt de mettre au point un produit efficace sur l'ensemble de ces critères nécessitant le moins de prises possibles.

La demanderesse a découvert de manière surprenante que l'association d'extrait de thé vert, de vitamine C et de composés métalliques choisis parmi le zinc, le chrome, le fer, et le cuivre, dans des compléments alimentaires ou des compositions nutraceutiques, permettait d'exercer une action simultanée sur les trois cibles suivantes : peau, phanères (cheveux, ongles) et poids, en administrant des doses relativement faibles de produits et un nombre réduit de prises quotidiennes. En outre, la demanderesse a découvert une manière d'administrer ces différents produits sans qu'aucune incompatibilité ne se manifeste.

Par ailleurs, l'extrait de thé vert, la vitamine C et les composés métalliques choisis parmi le zinc, le chrome, le fer, et le cuivre selon la présente invention, s'avèrent être des composés cosmétiquement, et nutraceutiquement acceptables, non agressifs, ni toxiques, ni irritants pour la peau, hypoallergéniques, apaisants, hydratants et anti-inflammatoires pour la peau. De plus, ces composés sont obtenus par des procédés d'extraction classique, et sont disponibles commercialement.

La présente invention a ainsi pour objet une composition, destinée à être administrée par voie orale, comprenant :
- au moins une première composition (a) contenant un extrait de thé vert et de la vitamine C, et éventuellement au moins un composé métallique choisi parmi le zinc, le chrome et un mélange de ceux-ci, et
- au moins une seconde composition (b) contenant au moins un composé métallique choisi parmi le fer, le cuivre, le chrome et un mélange de ceux-ci, ladite seconde composition (b) ne contenant pas de zinc et
lesdits composés métalliques des compostions (a) et (b) étant avantageusement sous forme de sels ou de complexes,
comme produit de combinaison pour une utilisation séparée et étalée dans le temps, dans le traitement cosmétique du corps humain.

Avantageusement selon la présente invention, la vitamine C d'une part et le fer et le cuivre d'autre part ne sont pas administrés simultanément dans la même composition afin d'éviter tout risque d'interférence de leurs activités pro et anti-oxydantes. Par ailleurs, le zinc et le fer ne sont pas administrés simultanément dans la même composition afin d'éviter tout risque de compétition d'absorption digestive de ces deux éléments.

Dans un mode de réalisation particulier de la présente invention, l'extrait de thé vert est un extrait de feuilles de thé vert, avantageusement sous forme de poudre. Cet extrait est classiquement obtenu par macération de thé vert dans un solvant pouvant être l'eau, un solvant type éthanol, hexane, ou avantageusement un mélange hydro-alcoolique, puis transformation en poudre par atomisation.

Dans un autre mode de réalisation particulier de la présente invention, la vitamine C se présente sous forme d'acide L-ascorbique, de L-ascorbate de sodium, L-ascorbate de calcium, de L-ascorbate de potassium ou de L-ascorbyle 6-palmitate.

Dans un autre mode de réalisation particulier de la présente invention, les composés métalliques se présentent sous forme de poudre. Le zinc se présente avantageusement sous forme de sel de zinc, tel que l'acétate de zinc, le chlorure de zinc, le citrate de zinc, le gluconate de zinc, le lactate de zinc, l'oxyde de zinc, le carbonate de zinc ou le sulfate de zinc. Le zinc peut également se présenter sous forme d'un complexe zinc-lysine, zinc-glycine, ou zinc-méthionine.

Le chrome se présente avantageusement sous forme de sel de chrome, tel que le chlorure de chrome (III) ou le sulfate de chrome (III). Le chrome peut également se présenter sous forme d'un complexe chrome-lysine, chrome-glycine, ou chrome-méthionine. Le fer se présente avantageusement sous forme de sel de fer, tel que le carbonate ferreux, le citrate ferreux, le citrate ferrique d'ammonium, le gluconate ferreux, le fumarate ferreux, le diphosphate ferrique de sodium, le lactate ferreux, le sulfate ferreux, le diphosphate ou pyrophosphate ferrique, ou le saccharate ferrique. Le fer peut également se présenter sous forme de fer élémentaire (issu de la réduction du carbonyle, de la réduction électrolytique et de la réduction de l'hydrogène), ou d'un complexe fer-lysine, fer-glycine, ou fer-méthionine. Le cuivre se présente avantageusement sous forme de sel de cuivre, tel que le carbonate de cuivre, le citrate de cuivre, le gluconate de cuivre, ou le sulfate de cuivre. Le cuivre peut également se présenter sous forme d'un complexe cuivre-lysine, cuivre-glycine, ou cuivre-méthionine.

Par complexe métallique, on entend au sens de la présente invention un composé métallique tel que le zinc, le chrome, le fer ou le cuivre en association avec un autre composé du type acide aminé, levure, ou protéine. Parmi les acides aminés pouvant être utilisés pour former un complexe métallique, peuvent être cités la lysine, la glycine et la méthionine.

Avantageusement, la composition selon la présente invention est destinée à traiter les affections et/ou déséquilibres de la peau, des phanères et/ou à traiter la surcharge pondérale. Ainsi, la composition peut agir sur l'une des trois cibles suivantes : peau, phanère, poids, ou sur l'ensemble de ces trois cibles simultanément.

De manière avantageuse, la composition selon la présente invention peut agir sur l'amélioration de l'aspect de la peau, en particulier sur l'hydratation de la peau, sur l'amélioration de la densité cutanée (réduction de l'apparence de peau « flétrie »), sur l'élasticité et sur la fermeté de la peau, ainsi que sur la réduction de la profondeur des rides. La composition selon la présente invention peut également agir sur la réduction de l'aspect peau d'orange de la peau (cellulite). De manière avantageuse, la composition selon la présente invention peut également agir au niveau des cheveux, notamment sur la réduction de la chute des cheveux, l'amélioration de leur résistance, de leur volume et de leur brillance, ainsi qu'au niveau des ongles, notamment sur l'amélioration de leur dureté et sur une réduction de la fréquence de leur casse. Enfin, la composition selon la présente invention peut avantageusement agir sur l'amélioration de la silhouette et la surcharge pondérale, en particulier sur la réduction de poids, et sur la réduction du volume de zones d'accumulation de graisses, notamment au niveau des cuisses et des hanches.

Avantageusement selon la présente invention, la composition (a) contient au moins un extrait de thé vert, de la vitamine C, un sel de zinc et un sel de chrome, et la composition (b) contient au moins un sel de fer et un sel de cuivre.

Selon une caractéristique particulière de la présente invention, l'extrait de thé vert est présent à une concentration comprise entre 1 et 50 % en poids, avantageusement entre 2 et 50 % en poids, encore plus avantageusement entre 5 et 30 % en poids, encore plus avantageusement entre 10 et 30 % en poids, par rapport au poids total de la composition (a).

Selon une autre caractéristique particulière de la présente invention, la vitamine C est présente à une concentration comprise entre 0,5 et 50 % en poids, avantageusement entre 2 et 50 % en poids, encore plus avantageusement entre 2 et 30 % en poids, encore plus avantageusement entre 2 et 15 % en poids, par rapport au poids total de la composition (a).

Selon une autre caractéristique particulière de la présente invention, le zinc est présent à une concentration comprise entre 0 et 3 % en poids, avantageusement entre 0,2 et 3 % en poids, encore plus avantageusement entre 0,2 et 1 % en poids, encore plus avantageusement entre 0,2 et 0,5 % en poids, par rapport au poids total de la composition (a) et/ou le chrome est présent à une concentration comprise entre 0 et 1 % en poids, avantageusement entre 0 et 0,05 % en poids, encore plus avantageusement entre 0,0002 et 0,04 % en poids, encore plus avantageusement entre 0,001 et 0,04 % en poids, par rapport au poids total de la composition (a) ou de la composition (b).

Selon une autre caractéristique particulière de la présente invention, le fer est présent à une concentration comprise entre 0 et 10% en poids, avantageusement entre 0,2 et 10 % en poids, encore plus avantageusement entre 0,2 et 3 % en poids, par rapport au poids total de la composition (b) et/ou le cuivre est présent à une concentration comprise entre 0 et 1% en poids, avantageusement entre 0,02 et 1 % en poids, encore plus avantageusement entre 0,02 et 0,5 % en poids, encore plus avantageusement entre 0,02 et 0,2 % en poids, par rapport au poids total de la composition (b).

La composition selon la présente invention est avantageusement une composition nutraceutique ou un complément alimentaire. Dans un mode de réalisation particulier de la présente invention, les compositions (a) et (b) se présentent sous forme de capsules, gélules, comprimés, granules, poudres ou solutions buvables. Lorsque les compositions (a) et (b) se présentent sous forme de capsules molles ou de gélules, l'enveloppe de ces capsules molles ou de ces gélules peut contenir notamment de la gélatine animale telle que la gélatine de poisson, de la glycérine, ou un matériau d'origine végétale tel qu'un dérivé de cellulose ou d'amidon, ou une protéine végétale. Lorsque les compositions se présentent sous forme de gélules, de comprimés, ou de granules, le mélange d'actifs peut être fixé sur un support pulvérulent tel que la silice, la cellulose, et la maltodextrine.

Avantageusement selon la présente invention, les compositions (a) et (b) se présentent toutes deux sous forme de capsules molles ou de gélules et l'extrait de thé vert est présent à une concentration comprise entre 10 et 30 % en poids, par rapport au poids total de la composition (a), la vitamine C est présente à une concentration comprise entre 2 et 15 % en poids, par rapport au poids total de la composition (a), le zinc est présent à une concentration comprise entre 0,2 et 0,5 % en poids, par rapport au poids total de la composition (a), le chrome est présent à une concentration comprise entre 0,001 et 0,04 % en poids, par rapport au poids total de la composition (a), le fer est présent à une concentration comprise entre 0,2 et 10 % en poids, par rapport au poids total de la composition (b), et le cuivre est présent à une concentration comprise entre 0,02 et 0,2 % en poids, par rapport au poids total de la composition (b). Lorsque les compositions (a) et (b) se présentent toutes deux sous forme de capsules molles ou de gélules, on entend « par poids total de la composition » au sens de la présente invention, le poids du contenu des capsules ou gélules ajouté au poids de l'enveloppe desdites capsules ou gélules.

Avantageusement selon la présente invention, la composition est titrée de manière à permettre l'administration d'une dose journalière de 100 à 3000 mg, de préférence de 200 à 2000 mg, encore plus préférentiellement de 300 à 1500 mg, d'extrait de thé vert ; de 50 à 1000 mg, de préférence de 100 à 500 mg, de vitamine C ; de 0 à 50 mg, de préférence de 1 à 50 mg, encore plus préférentiellement de 5 à 20 mg, de zinc ; de 0 à 300 µg, de préférence de 10 à 300 µg, encore plus préférentiellement de 20 à 100 µg, de chrome ; de 0 à 100 mg, de préférence de 1 à 100 mg, encore plus préférentiellement de 5 à 50 mg, de fer ; de 0 à 20 mg, de préférence de 0,5 à 20 mg, encore plus préférentiellement de 1 à 10 mg, de cuivre.

Avantageusement selon la présente invention, la composition est une composition ou un complément nutraceutique ou alimentaire, et peut comprendre tout véhicule ou excipient approprié, acceptable du point de vue cosmétique ou nutraceutique, ainsi que des additifs conventionnels, connus de l'homme du métier.

Avantageusement selon la présente invention, la composition contient d'autres ingrédients actifs qui peuvent compléter ou renforcer l'action des ingrédients principaux, à savoir des vitamines tels que la provitamine A, les caroténoïdes, les vitamines du groupe B, notamment les vitamines B2, B5, 86 et B8 ; d'autres sels métalliques tels que les sels de sélénium ou de manganèse ; des acides gras insaturés tels que l'huile de bourrache ou l'huile de poissons ; un extrait de marc de raisins ; ou encore du cartilage de requin.

La présente invention a également pour objet un procédé de traitement cosmétique du corps humain, **caractérisé en ce qu**'il implique l'administration par voie orale d'un produit de combinaison comprenant :
- au moins une première composition (a) contenant un extrait de thé vert et de la vitamine C, et éventuellement au moins un composé métallique choisi parmi le zinc, le chrome et un mélange de ceux-ci, et
- au moins une seconde composition (b) contenant au moins un composé métallique choisi parmi le fer, le cuivre, le chrome et un mélange de ceux-ci, ladite seconde composition (b) ne contenant pas de zinc et
lesdits composés métalliques des compostions (a) et (b) étant avantageusement sous forme de sels ou de complexes,
pour une utilisation séparée et étalée dans le temps.

Le procédé selon l'invention est avantageusement destiné à traiter les affections et/ou déséquilibres de la peau, des phanères et/ou à traiter la surcharge pondérale. Ainsi, la composition selon l'invention (produit de combinaison) peut agir sur l'une des trois cibles suivantes : peau, phanère, poids, ou sur l'ensemble de ces trois cibles simultanément.

Avantageusement selon la présente invention, la composition (a) contient au moins un extrait de thé vert, de la vitamine C, un sel de zinc et un sel de chrome, et la composition (b) contient au moins un sel de fer et un sel de cuivre.

Dans un mode de réalisation particulier du procédé de la présente invention, les différents composés des compositions (a) et (b) présentent les mêmes concentrations que celles mentionnées ci-dessus.

Selon une caractéristique particulière de la présente invention, le procédé implique l'administration d'une dose journalière d'extrait de thé vert comprise entre 100 et 3000 mg, avantageusement entre 200 et 2000 mg, encore plus avantageusement entre 300 et 1500 mg.

Selon une autre caractéristique particulière de la présente invention, le procédé implique l'administration d'une dose journalière de vitamine C comprise entre 50 et 1000 mg, avantageusement entre 60 et 500 mg, encore plus avantageusement entre 100 et 500 mg.

Selon une autre caractéristique particulière de la présente invention, le procédé implique l'administration d'une dose journalière de zinc comprise entre 0 et 50 mg, avantageusement entre 1 et 50 mg, encore plus avantageusement entre 5 et 20 mg, et/ou d'une dose journalière de chrome comprise entre 0 et 300 µg, avantageusement entre 10 et 300 µg, encore plus avantageusement entre 12 et 100 µg, encore plus avantageusement entre 20 et 100 µg.

Selon une autre caractéristique particulière de la présente invention, le procédé implique l'administration d'une dose journalière de fer comprise entre 0 et 100 mg, avantageusement entre 1 et 100 mg, encore plus avantageusement entre 5 et 50 mg, encore plus avantageusement entre 10 et 50 mg, et/ou d'une dose journalière de cuivre comprise entre 0 et 20 mg, avantageusement entre 0,5 et 20 mg, encore plus avantageusement entre 1 et 10 mg.

Avantageusement selon la présente invention, la composition (a) est administrée en première partie de journée, notamment le matin ou à midi, et la composition (b) est administrée en seconde partie de journée, notamment au cours de l'après-midi ou le soir. Les actifs stimulants de la composition (a), à savoir le thé vert et la vitamine C, sont ainsi avantageusement administrés en première partie de journée pour éviter des difficultés d'endormissement le soir.

Dans un mode de réalisation particulier de la présente invention, les compositions (a) et (b) se présentent toutes deux sous forme de capsules molles. Avantageusement selon la présente invention, la prise de deux capsules de composition (a) le matin et d'une capsule de composition (b) le soir permettent d'apporter une dose efficace de chacun des ingrédients nécessaires pour agir sur l'ensemble des trois cibles peau, phanères et poids, tout en séparant les actifs incompatibles entre eux.

Les exemples suivants sont destinés à illustrer l'invention sans aucunement en limiter la portée. A moins qu'il n'en soit précisé autrement, les pourcentages indiqués dans les exemples suivants sont des pourcentages en poids.

### Exemples de compositions selon la présente invention et de leurs procédés de préparation :

### Exemple 1 : Compositions (a) et (b) se présentant sous forme de capsules molles - procédé de préparation

### Composition (a) :

| INGREDIENTS | QUANTITES PAR CAPSULE |
|---|---|
| Huile de Bourrache | 357 mg |
| | |
| Extrait de Thé vert | 200 mg |
| | |
| Ascorbate de calcium (Vitamine C) | 75 mg |
| | |
| Bétacarotène naturel (Vitamine A) | 2,4 mg |
| | |
| Lécithine | 13,6 mg |
| | |
| Cire d'abeille jaune | 10,8 mg |
| | |
| Chlorure de Chrome | 0,06 mg |
| | |
| Sulfate de Zinc | 13,7 mg |
| | |
| Sélénate de sodium | 0,05 mg |
| | |
| Enveloppe de la capsule | Gélatine de poisson, Glycérine, Eau et colorants alimentaires |

### Composition (b) :

| INGREDIENTS | QUANTITES PAR CAPSULE |
|---|---|
| Huile de poisson concentrée | 365 mg |
| | |
| Extrait de Marc de raisin | 150 mg |
| | |
| Cartilage de requin | 100 mg |
| | |
| Fumarate de fer | 31,8 mg |
| | |
| Sulfate de Cuivre | 2,6 mg |
| | |
| Cire jaune d'abeille | 15 mg |
| | |
| Vitamine B2 (Riboflavin) | 1,6 mg |
| | |
| Vitamine B5 (pantothénate de calcium) | 6,55 mg |
| | |
| Vitamine B6 (chlorhydrate de pyridoxine) | 2,4 mg |
| | |
| Vitamine B8 (biotine) | 0,15 mg |
| | |
| Lécithine | 12 mg |
| | |
| Enveloppe de la capsule | Gélatine de poisson, Glycérine, Eau et colorants alimentaires |

### Procédé de préparation des compositions (a) et (b) :

Le procédé consiste à disperser sous agitation la lécithine et la cire d'abeille dans l'huile majoritaire chauffée (bourrache pour composition (a) et huile de poisson pour composition (b)), puis à incorporer, toujours sous agitation, les différents ingrédients dans le mélange précédent.

Ce mélange est ensuite encapsulé dans une enveloppe constituée de gélatine, de glycérine, d'eau et de colorants par l'intermédiaire d'une machine spécifique pour ce type d'opération (machine de fabrication des capsules molles), puis les capsules molles sont séchées (élimination d'une partie de l'eau de l'enveloppe).

### Exemple 2 : Evaluation de l'activité induite par l'administration de compositions salon la présente invention sur les quatre cibles : peau - cheveux - ongles - poids

Les compositions (a) et (b) de l'exemple 1 ont été administrées à des volontaires à raison de 2 capsules de composition (a) le matin et de 1 capsule de composition (b) le soir pendant 2 mois. Les volontaires, de sexe féminin et âgées de plus de 40 ans, ont été sélectionnées car elles répondaient au moins à un des critères suivants :
- chute régulière de cheveux depuis plus d'une semaine (méthode de collecte des cheveux tombés par le volontaire),
- rides du visage (pâte d'oie) ayant une profondeur d'au moins 0,3 mm par mesure profilométrique (empreinte de silicone),
- sécheresse cutanée objective au niveau des jambes par un questionnaire complété par les volontaires,
- ongles cassants nécessitant une coupe très régulière,
- surpoids défini par un IMC (Indice de Masse Corporelle) supérieur à 25.

Les résultats observés ont été les suivants à l'issue des 2 mois de traitement :
- chute des cheveux, diminuée de 32 %,
- réduction de 40 % de la profondeur des rides,
- amélioration de la sécheresse cutanée chez 73 % des volontaires,
- amélioration de la solidité des ongles chez 64 % des volontaires,
- perte de poids de 5,1 kg en moyenne.

### Exemple 3 : Evaluation clinique de l'activité induite par l'administration de compositions selon la présente invention sur les quatre cibles : peau - cheveux - ongles, - poids

Les compositions suivantes ont été administrées à des femmes à raison de 2 capsules le matin et une capsule le soir. Le terme d'« AJR » signifie « Apports Journaliers Recommandés ».

Capsules pour le matin :

| Ingrédients | Pour 2 capsules | %AJR |
|---|---|---|
| Extrait aqueux de Thé vert | 400 mg | |
| Vitamine C | 120 mg | 200 % |
| Bétacarotène naturel | 800 µg ER | 100 % |
| Zinc | 10 mg | 67 % |
| Sélénium | 40 µg | 80 % |
| Chrome | 25 µg | 100% |
| Huile de Bourrache | 700 mg | |

Capsule pour le soir :

| Ingrédients | Pour 1 capsule | % AJR |
|---|---|---|
| Extrait de raisin | 150 mg | |
| Cartilage de requin | 100 mg | |
| Vitamine B2 | 1,6 mg | 100 % |
| Vitamine B5 | 6 mg | 100 % |
| Vitamine B6 | 2 mg | 100 % |
| Vitamine B8 | 150 µg | 100 % |
| Cuivre | 1 mg | 50 % |
| Fer | 10 mg | 71 % |
| Huile de poisson | 350 mg | |

### 3.1. Etude perte de cheveux

Cette étude a été réalisée auprès de 30 femmes de plus de 30 ans (âge moyen 48,8 ans ; mini : 35ans, maxi : 67ans) et présentant une chute anormale de cheveux depuis plusieurs mois. L'évaluation de l'efficacité était basée sur une technique expérimentalement validée de recueil des cheveux perdus au cours du brossage.

Chaque femme devait procéder à un brossage standardisé de ses cheveux secs une fois par jour, recueillir les cheveux tombés et recueillis par la brosse, et les introduire dans un flacon plastique préalablement pesé. Ce recueil quotidien des cheveux était réalisé pendant le mois précédant le début du traitement et pendant tout le 2^{ème} mois de traitement avec les compositions selon l'invention mentionnées ci-dessus.

Les résultats démontrent une activité réelle des compositions selon l'invention sur la perte des cheveux, puisque la moyenne journalière de chute de cheveux était initialement de 52 mg par jour, et était seulement de 21,6 mg par jour au cours du deuxième mois de prise des capsules, soit une diminution très importante de plus de 50%.

Cette évaluation très objective obtenue par la pesée des cheveux par l'investigation a été confirmée par les volontaires : 85% des femmes ont observé une diminution nette de leur perte de cheveux.

### 3.2. Etude amincissement

Cette étude a été réalisée auprès de 22 femmes de plus de 30 ans (moyenne d'âge : 48,3ans) présentant impérativement une surcharge pondérale définie par un rapport : Poids (kg) / Taille (cm) ² compris entre 25 et 30 (moyenne 27,8).

L'étude a été réalisée pendant 2 mois, chaque volontaire prenant chaque jour 2 capsules le matin et 1 capsule le soir, telles que décrites ci-dessus. Le protocole imposait aux volontaires de ne rien changer à leurs habitudes alimentaires pendant la durée de l'étude et, en particulier, de ne pas commencer ou poursuivre un régime hypocalorique amincissant.

Chaque volontaire était vue 3 fois par le médecin : avant le début de l'étude, après 1 mois de prise, et après 2 mois de prise. Les critères évalués étaient le poids, la masse grasse, mesurée par une balance impédance métrique, et les périmètres des cuisses et de l'abdomen, mesurés par une technique reproductible assurant que les mesures pour chaque patiente à chaque visite étaient réalisées exactement au même niveau.

Les résultats ont été les suivants :
- poids : une perte de poids statiquement significative était observée dès le premier mois, et atteignait 1,2 kg après 2 mois de prise,
- masse grasse : une diminution statistiquement significative de la masse grasse était observée dès le premier mois, et atteignait 0,882 kg après 2 mois de prise,
- périmètre des cuisses : diminution très significative dès le premier mois (0,9 cm) qui se poursuivait au cours du deuxième mois, pour atteindre 1,3 cm, et
- périmètre de l'abdomen : une diminution statiquement significative était observée dès le premier mois (1 cm), et se poursuivait de façon importante au cours du deuxième mois (1,9 cm).

### 3.3. Etude rides

Parmi les 52 femmes ayant participé à l'une des deux études (« amincissement » ou « cheveux »), 17 présentaient à l'inclusion au moins une ride assez marquée au niveau de la patte d'oie. Pour ces 17 femmes, une empreinte au silicone était prise avant et à la fin de l'étude.

L'étude a duré 2 mois. Les femmes ont pris les capsules quotidiennement pendant toute la durée de l'étude.

Ces empreintes étaient ensuite analysées par profilométrie laser permettant de mesurer les dimensions (longueur, largeur et profondeur) exactes de la ride au micromètre près. Cette analyse profilométrique de l'empreinte permettait de calculer plusieurs paramètres :
- complexité : c'est le ratio entre la surface cutanée développée et la projection horizontale de cette surface.
- profondeur : profondeur moyenne de la ride dans la surface analysée.
- volume : volume moyen de la ride dans la surface analysée.

Les résultats obtenus ont été très intéressants :
- la complexité moyenne est passée de 24,5 % à 17,6 %, soit une diminution de 25 %,
- la profondeur moyenne est passée de 320 µm à 213 µm, soit une diminution de 27 %,
- le volume moyen est passé de 0,595 mm³ à 0,400 mm³, soit une diminution de 23 %.

L'étude a ainsi permis de montrer que l'administration de compositions selon l'invention permettait de diminuer significativement les rides en termes de visibilité.

### 3.4. Etude hydratation cutanée

La mesure du niveau d'hydratation cutanée au niveau de la face externe des jambes a été réalisée par un cornéomètre avant et après l'étude, sur l'ensemble des 52 femmes.

Le niveau moyen d'hydratation cutanée était de 54,47 avant l'étude, de 56,73 après 1 mois, et de 61,71 après 2 mois. Cette amélioration de l'hydratation cutanée a donc été statistiquement très significative dès le premier mois d'utilisation.

### 3.5. Etude ongles

Sur l'ensemble de 52 femmes ayant participé à l'une des deux études amincissement ou cheveux, 26 femmes se plaignaient, au moment du recrutement, de problèmes d'ongles. Les 2 problèmes les plus souvent rencontrés étaient les ongles qui se dédoublent et les ongles cassants.

Après 2 mois de prise des capsules selon l'invention, 70% des femmes ont noté une amélioration nette, et souvent spectaculaire, de la qualité de leurs ongles.

En conclusion, les compositions selon l'invention permettent d'engendrer une perte de poids et d'améliorer la silhouette, de diminuer la perte des cheveux, de diminuer les rides, d'améliorer l'hydratation cutanée, et/ou d'améliorer la qualité des ongles, notamment les ongles cassants.

## Revendications

1. Procédé de traitement cosmétique du corps humain visant à améliorer l'aspect esthétique de la peau et/ou des phanères et/ou de la silhouette, **caractérisé en ce qu'**il implique l'administration par voie orale d'un produit de combinaison comprenant :
- une première composition (a) contenant un extrait de thé vert et de la vitamine C, et éventuellement au moins un composé métallique choisi parmi le zinc, le chrome et un mélange de ceux-ci, et
- une seconde composition (b) contenant au moins un composé métallique choisi parmi le fer, le cuivre, le chrome et un mélange de ceux-ci, ladite seconde composition (b) ne contenant pas de zinc,
lesdits composés métalliques des compostions (a) et (b) étant avantageusement sous forme de sels ou de complexes,
pour une utilisation séparée et étalée dans le temps.

2. Procédé de traitement cosmétique selon la revendication 1, **caractérisé en ce que** la composition (a) contient au moins un extrait de thé vert, de la vitamine C, un sel de zinc et un sel de chrome, et la composition (b) contient au moins un sel de fer et un sel de cuivre.

3. Procédé de traitement cosmétique selon la revendication 1 ou 2, **caractérisé en ce que** l'extrait de thé vert est présent à une concentration comprise entre 2 et 50 % en poids, avantageusement entre 10 et 30 % en poids, par rapport au poids total de la composition (a).

4. Procédé de traitement cosmétique selon l'une quelconque des revendications 1 à 3, **caractérisé en ce que** la vitamine C est présente à une concentration comprise entre 0,5 et 50 % en poids, avantageusement entre 2 et 15 % en poids, par rapport au poids total de la composition (a).

5. Procédé de traitement cosmétique selon l'une quelconque des revendications précédentes, **caractérisé en ce que** le zinc est présent à une concentration comprise entre 0 et 3 % en poids, avantageusement entre 0,2 et 1 % en poids, par rapport au poids total de la composition (a) et/ou le chrome est présent à une concentration comprise entre 0 et 1 % en poids, avantageusement entre 0 et 0,05 % en poids, par rapport au poids total de la composition (a) ou de la composition (b).

6. Procédé de traitement cosmétique selon l'une quelconque des revendications précédentes, **caractérisé en ce que** le fer est présent à une concentration comprise entre 0 et 10 % en poids, avantageusement entre 0,2 et 3 % en poids, par rapport au poids total de la composition (b) et/ou le cuivre est présent à une concentration comprise entre 0 et 1 % en poids, avantageusement entre 0,02 et 0,5 % en poids, par rapport au poids total de la composition (b).

7. Procédé de traitement cosmétique selon l'une quelconque des revendications précédentes, **caractérisé en ce qu'**il implique l'administration d'une dose journalière d'extrait de thé vert comprise entre 100 et 3000 mg, avantageusement entre 200 et 2000 mg.

8. Procédé de traitement cosmétique selon l'une quelconque des revendications précédentes, **caractérisé en ce qu'**il implique l'administration d'une dose journalière de vitamine C comprise entre 50 et 1000 mg, avantageusement entre 60 et 500 mg, typiquement entre 100 et 500 mg.

9. Procédé de traitement cosmétique selon l'une quelconque des revendications précédentes, **caractérisé en ce qu'**il implique l'administration d'une dose journalière de zinc comprise entre 0 et 50 mg, avantageusement entre 1 et 50 mg, typiquement entre 5 et 20 mg, et/ou d'une dose journalière de chrome comprise entre 0 et 300 µg, avantageusement entre 10 et 300 µg, typiquement entre 20 et 100 µg.

10. Procédé de traitement cosmétique selon l'une quelconque des revendications précédentes, **caractérisé en ce qu'**il implique l'administration d'une dose journalière de fer comprise entre 0 et 100 mg, avantageusement entre 5 et 50 mg, et/ou d'une dose journalière de cuivre comprise entre 0 et 20 mg, avantageusement entre 1 et 10 mg.

11. Procédé de traitement cosmétique selon l'une quelconque des revendications précédentes, **caractérisé en ce que** la composition (a) est administrée en première partie de journée et la composition (b) est administrée en seconde partie de journée.

12. Procédé de traitement cosmétique selon l'une quelconque des revendications précédentes, **caractérisé en ce que** les compositions (a) et (b) se présentent sous forme de capsules, gélules, comprimés, granules, poudres ou solutions buvables.

## Claims

1. Method for cosmetic treatment of the human body, intended to enhance the aesthetic appearance of the skin and/or of the skin appendages and/or of the figure, **characterised in that** it involves the oral administration of a combination product comprising:
- a first composition (a) containing a green tea extract, vitamin C, and optionally at least one metallic compound selected from zinc, chromium and a mixture thereof, and
- a second composition (b) containing at least one metallic compound selected from iron, copper, chromium and a mixture thereof, said second composition (b) being free of zinc,
wherein said metallic compounds of compositions (a) and (b) are advantageously in the form of salts or complexes,
for separate and consecutive administration.

2. Method for cosmetic treatment according to claim 1, **characterised in that** composition (a) contains at least one green tea extract, vitamin C, a zinc salt and a chromium salt, and composition (b) contains at least one iron salt and one copper salt.

3. Method for cosmetic treatment according to claim 1 or 2, **characterised in that** the green tea extract is present at a concentration of between 2 and 50 % by weight, and advantageously between 10 and 30 % by weight, with respect to the total weight of composition (a).

4. Method for cosmetic treatment according to any one of claims 1 to 3, **characterised in that** the vitamin C is present at a concentration of between 0.5 and 50 % by weight, and advantageously between 2 and 15 % by weight, with respect to the total weight of composition (a).

5. Method for cosmetic treatment according to any one of the previous claims, **characterised in that** the zinc is present at a concentration of between 0 and 3 % by weight, and advantageously between 0.2 and 1 % by weight, with respect to the total weight of composition (a) and/or the chromium is present at a concentration of between 0 and 1 % by weight, and advantageously between 0 and 0.05 % by weight, with respect to the total weight of composition (a) or composition (b).

6. Method for cosmetic treatment according to any one of the previous claims, **characterised in that** the iron is present at a concentration of between 0 and 10 % by weight, and advantageously between 0.2 and 3 % by weight, with respect to the total weight of composition (b) and/or the copper is present at a concentration of between 0 and 1 % by weight, and advantageously between 0.02 and 0.5 % by weight, with respect to the total weight of composition (b)

7. Method for cosmetic treatment according to any one of the previous claims, **characterised in that** it involves the administration of a daily dose of green tea extract of between 100 and 3000 mg, and advantageously between 200 and 2000 mg.

8. Method for cosmetic treatment according to any one of the previous claims, **characterised in that** it involves the administration of a daily dose of vitamin C of between 50 and 1000 mg, advantageously between 60 and 500 mg, and more advantageously between 100 and 500 mg.

9. Method for cosmetic treatment according to any one of the previous claims, **characterised in that** it involves the administration of a daily dose of zinc of between 0 and 50 mg, advantageously between 1 and 50 mg, more advantageously between 5 and 20 mg, and/or a daily dose of chromium of between 0 and 300 µg, advantageously between 10 and 300 µg, more advantageously between 20 and 100 µg.

10. Method for cosmetic treatment according to any one of the previous claims, **characterised in that** it involves the administration of a daily dose of iron of between 0 and 100 mg, advantageously between 5 and 50 mg, and/or a daily dose of copper of between 0 and 20 mg, advantageously between 1 and 10 mg.

11. Method for cosmetic treatment according to any one of the previous claims, **characterised in that** composition (a) is administered in the first part of the day and composition (b) is administered in the second part of the day.

12. Method for cosmetic treatment according to any one of the previous claims, **characterised in that** compositions (a) and (b) are in the form of capsules, gel caps, tablets, pellets, powders or drinkable solutions.

## Patentansprüche

1. Verfahren zur kosmetischen Behandlung des menschlichen Körpers mit dem Zweck, den ästhetischen Eindruck der Haut und/oder der Hautanhangsgebilde und/oder der Körperkonturen zu verbessern, **dadurch gekennzeichnet, dass** es die orale Verabreichung eines Kombinationserzeugnisses einschließt, das enthält:
- eine erste Zusammensetzung (a), die einen Extrakt aus grünem Tee und Vitamin C und möglicherweise zumindest eine metallische Verbindung ausgewählt aus Zink, Chrom und einer Mischung aus diesen umfasst, und
- eine zweite Zusammensetzung (b), die zumindest eine metallische Verbindung ausgewählt aus Eisen, Kupfer, Chrom und einer Mischung aus diesen umfasst, wobei die besagte zweite Zusammensetzung (b) kein Zink umfasst, wobei die metallischen Verbindungen der Zusammensetzungen (a) und (b) vorteilhafterweise in der Form von Salzen oder von Komplexen vorliegen,
zur getrennten und über die Zeit verteilten Anwendung.

2. Verfahren zur kosmetischen Behandlung nach Anspruch 1, **dadurch gekennzeichnet, dass** die Zusammensetzung (a) zumindest einen Extrakt aus grünem Tee, Vitamin C, ein Zinksalz und ein Chromsalz umfasst und die Zusammensetzung (b) zumindest ein Eisensalz und ein Kupfersalz umfasst.

3. Verfahren zur kosmetischen Behandlung nach Anspruch 1 oder 2, **dadurch gekennzeichnet, dass** der Extrakt des grünen Tees in einer Konzentration zwischen 2 und 50 Gew.-%, vorteilhafterweise zwischen 10 und 30 Gew.-%, bezogen auf das Gesamtgewicht der Zusammensetzung (a), vorliegt

4. Verfahren zur kosmetischen Behandlung nach einem der Ansprüche 1 bis 3, **dadurch gekennzeichnet, dass** das Vitamin C in einer Konzentration zwischen 0,5 und 50 Gew.-%, vorteilhafterweise zwischen 2 und 15 Gew.-%, bezogen auf das Gesamtgewicht der Zusammensetzung (a), vorliegt.

5. Verfahren zur kosmetischen Behandlung nach einem der vorhergehenden Ansprüche, **dadurch gekennzeichnet, dass** das Zink in einer Konzentration zwischen 0 und 3 Gew.-%, vorteilhafterweise zwischen 0,2 und 1 Gew.-%, bezogen auf das Gesamtgewicht der Zusammensetzung (a), vorliegt, und/oder das Chrom in einer Konzentration zwischen 0 und 1 Gew.-%, vorteilhafterweise zwischen 0 und 0,05 Gew.-%, bezogen auf das Gesamtgewicht der Zusammensetzung (a) oder der Zusammensetzung (b), vorliegt.

6. Verfahren zur kosmetischen Behandlung nach einem der vorhergehenden Ansprüche, **dadurch gekennzeichnet, dass** das Eisen in einer Konzentration zwischen 0 und 10 Gew.-%, vorteilhafterweise zwischen 0,2 und 3 Gew.-%, bezogen auf das Gesamtgewicht der Zusammensetzung (b), vorliegt, und/oder das Kupfer in einer Konzentration zwischen 0 und 1 Gew.-%, vorteilhafterweise zwischen 0,02 und 0,5 Gew.-%, bezogen auf das Gesamtgewicht der Zusammensetzung (b), vorliegt.

7. Verfahren zur kosmetischen Behandlung nach einem der vorhergehenden Ansprüche, **dadurch gekennzeichnet, dass** es die Verabreichung einer täglichen Dosis des Extrakts des grünen Tees zwischen 100 und 3000 mg, vorteilhafterweise zwischen 200 und 2000 mg, einschließt.

8. Verfahren zur kosmetischen Behandlung nach einem der vorhergehenden Ansprüche, **dadurch gekennzeichnet, dass** es die Verabreichung einer täglichen Dosis Vitamin C zwischen 50 und 1000 mg, vorteilhafterweise zwischen 60 und 500 mg, noch vorteilhafter zwischen 100 und 500 mg, einschließt.

9. Verfahren zur kosmetischen Behandlung nach einem der vorhergehenden Ansprüche, **dadurch gekennzeichnet, dass** es die Verabreichung einer täglichen Dosis Zink zwischen 0 und 50 mg, vorteilhafterweise zwischen 1 und 50 mg, noch vorteilhafter zwischen 5 und 20 mg, und/oder einer täglichen Dosis Chrom zwischen 0 und 300 µg, vorteilhafterweise zwischen 10 und 300 µg, noch vorteilhafter zwischen 20 und 100 µg, einschließt.

10. Verfahren zur kosmetischen Behandlung nach einem der vorhergehenden Ansprüche, **dadurch gekennzeichnet, dass** es die Verabreichung einer täglichen Dosis Eisen zwischen 0 und 100 mg, vorteilhafterweise zwischen 5 und 50 mg, und/oder einer täglichen Dosis Kupfer zwischen 0 und 20 mg, vorteilhafterweise zwischen 1 und 10 mg, einschließt.

11. Verfahren zur kosmetischen Behandlung nach einem der vorhergehenden Ansprüche, **dadurch gekennzeichnet, dass** die Zusammensetzung (a) im ersten Tagesabschnitt verabreicht wird und die Zusammensetzung (b) im zweiten Tagesabschnitt verabreicht wird.

12. Verfahren zur kosmetischen Behandlung nach einem der vorhergehenden Ansprüche, **dadurch gekennzeichnet, dass** die Zusammensetzungen (a) und (b) in der Form von Kapseln, Gelkapseln, Tabletten, Granulat, Pulvern oder trinkbaren Lösungen vorliegen.
